# EUROPEAN PATENT APPLICATION

(11) **EP 2 110 158 A1**
(43) Date of publication of application: **21.10.2009**
(21) Application number: 08702972.4
(22) Date of filing: 09.01.2008
(51) Int. Cl.: A61N 5/06, A61B 18/20, G02B 6/00, G02B 6/02, G02B 6/036, G02B 6/04

(54) **OPTICAL FIBER, OPTICAL FIBER DEVICE, AND BUNDLE FIBER**

(30) Priority: 08.02.2007 JP 2007029368
(71) Applicant: Fujikura, Ltd., Tokyo 135-8512 (JP); Trohopoulos, Panagiotis N., 54622 Thessaloniki (GR)
(72) Inventor: TSUMANUMA, Takashi, Sakura-shi, Chiba 285-8550 (JP); SHAMOTO, Naoki, Sakura-shi, Chiba 285-8550 (JP); TROHOPOULOS, Panagiotis, N., GR-54622, Thessaloniki (GR)
(74) Representative: Grünecker, Kinkeldey, Stockmair & Schwanhäusser Anwaltssozietät
(86) International application number: PCT/JP2008/050101
(87) International publication number: WO 2008/096561

(57) **Abstract**

An optical fiber, an optical fiber device, and an optical fiber bundle, which are applied to an organic tubular tissue, for the medical application for removing a thrombus part deposited on an internal wall of tubular tissue are provided.

The optical fiber (20) which can be inserted in a tubular tissue (15) in an organism comprises a glass core layer (11), a covering core layer (12) which covers the glass core layer (11), and a cladding layer (13) which covers the covering core layer (12) and includes a plurality of apertural areas (23) in a longitudinal direction of the optical fiber (20). The width of the apertural area (23) in the longitudinal direction of the optical fiber (20) is narrowly formed in an entrance part of the optical fiber (20), and is widely formed as to be distanced in the longitudinal direction of the optical fiber (20) from the entrance part. The optical fiber device applies such the optical fiber. The optical fiber bundle bundles a plurality of such the optical fibers.

## Description

### TECHNICAL FIELD

The present invention relates to an optical fiber, an optical fiber device, and an optical fiber bundle, and in particular, relates to an optical fiber, an optical fiber device, and an optical fiber bundle of a medical application, which are applied to an organic tubular tissue.

### BACKGROUND ART

In an organic tubular tissue represented by a cardiac coronary artery blood vessel etc., a thrombus layer adheres to the wall part. The thrombus layer is a layer of cholesterol including padding of a fat and many calcium ingredients. In recent years, as a method to improve obstruction by the thrombus of a coronary artery, etc., there are a recanalization method by a balloon called the percutaneous menstrual blood vessel coronary artery plasty or PTCA (Percutaneous Transluminal Coronary Angioplasty), and a semipermanent recanalization method called a stent. However, since there are the problems, such as the generation of a restenosis, it cannot become a perfect therapeutic method.

On the other hand, PDT (Photodynamic Therapy) is a therapy which the organism introduces a photosensitizer, illuminates with the light of the specific wavelength a part of a cancer which a medicine concentrated selectively using the medicine of the photosensitizer concentrating on the part of cancers, such as a stomach and a lung, selectively, and collapses the cancer cell. Moreover, PDD (Photodynamic Diagnosis) is a diagnosing method which illuminates with the light of the specific wavelength using the medicine of the photosensitizer concentrating on the part of cancers, such as the stomach and the lung, selectively, and grips a broadening range of the cancer cells, such as the stomach and the lung, from the fluorescence.

On the other hand, in the use of an optical fiber sensor as a surface treatment technology by an optical fiber, it is already disclosed in Patent Literature 1 about a method of forming a plurality of notches on the surface of the optical fiber.
Patent Literature 1: Published Japanese translations of PCT International Publication for Patent Application No. H08-511343
Although the Photodynamic Therapy (PDT) or the Photodynamic Diagnosis (PDD) is applied to the part of cancers, such as the stomach and the lung, as above-mentioned, the PDT or the PDD is not applied to a lesion region in the tubular tissue of an organic narrow diameter represented in a cardiac coronary artery blood vessel etc.

The object of the present invention is to provide an optical fiber, an optical fiber device, and an optical fiber bundle of the medical application for being applied to organic tubular tissue and treating the lesion region of tubular tissue.

### DISCLOSURE OF INVENTION

According to one aspect of the present invention, an optical fiber which can be inserted in a tubular tissue in an organism, the optical fiber comprising: a glass core layer; a covering core layer which covers the glass core layer; and a cladding layer which covers the covering core layer and includes a plurality of apertural areas in a longitudinal direction of the optical fiber, wherein a width of the apertural area in the longitudinal direction of the optical fiber is narrowly formed in an entrance part of the optical fiber, and is widely formed as to be distanced in the longitudinal direction of the optical fiber from the entrance part, is provided.

According to another aspect of the present invention, an optical fiber which can be inserted in a tubular tissue in an organism, the optical fiber comprising: a glass core layer; a covering core layer which covers the glass core layer; and a cladding layer which covers the covering core layer and includes a plurality of apertural areas having constant width in a longitudinal direction of the optical fiber, wherein an arrangement density of the apertural area increases as to be distanced in the longitudinal direction of the optical fiber from an entrance part of the optical fiber, is provided.

According to another aspect of the present invention, an optical fiber which can be inserted in a tubular tissue in an organism, the optical fiber comprising: a glass core layer; a covering core layer which covers the glass core layer; and a cladding layer which covers the covering core layer and includes a spiral apertural area in a longitudinal direction of the optical fiber, wherein an arrangement density of the apertural area increases as to be distanced in the longitudinal direction of the optical fiber from an entrance part of the optical fiber, is provided.

According to another aspect of the present invention, an optical fiber which can be inserted in a tubular tissue in an organism, the optical fiber comprising: a core layer; and a cladding layer which covers the core layer and includes a plurality of apertural areas having constant width in a longitudinal direction of the optical fiber, wherein an arrangement density of the apertural area increases as to be distanced in the longitudinal direction of the optical fiber from an entrance part of the optical fiber, is provided.

According to another aspect of the present invention, an optical fiber device comprising: a laser light source; and an optical fiber connected to the laser light source, the optical fiber comprising a glass core layer, a covering core layer which covers the glass core layer, and a cladding layer which covers the covering core layer and including a plurality of apertural areas in a longitudinal direction of the optical fiber, a width of the apertural area in the longitudinal direction of the optical fiber is narrowly formed in an entrance part of the optical fiber and is widely formed as to be distanced in the longitudinal direction of the optical fiber from the entrance part, the optical fiber which can be inserted in a tubular tissue in an organism, is provided.

According to another aspect of the present invention, an optical fiber device comprising: a laser light source; and an optical fiber connected to the laser light source, the optical fiber comprising a glass core layer, a covering core layer which covers the glass core layer, and a cladding layer which covers the covering core layer and includes a plurality of apertural areas having constant width in a longitudinal direction of the optical fiber, an arrangement density of the apertural area increases as to be distanced in the longitudinal direction of the optical fiber from an entrance part of the optical fiber, the optical fiber which can be inserted in a tubular tissue in an organism, is provided.

According to another aspect of the present invention, an optical fiber device comprising: a laser light source; and an optical fiber connected to the laser light source, the optical fiber comprising a glass core layer, a covering core layer which covers the glass core layer, and a cladding layer which covers the covering core layer and includes a spiral apertural area in a longitudinal direction of the optical fiber, an arrangement density of the apertural area increases as to be distanced in the longitudinal direction of the optical fiber from an entrance part of the optical fiber, the optical fiber which can be inserted in a tubular tissue in an organism, is provided.

According to another aspect of the present invention, an optical fiber bundle comprising a plurality of optical fibers which can be inserted in a tubular tissue in an organism, each the optical fiber comprising: a glass core layer; a covering core layer which covers the glass core layer; and a cladding layer which covers the covering core layer and including a plurality of apertural areas in a longitudinal direction of the optical fiber, wherein a width of the apertural area in the longitudinal direction of the optical fiber is narrowly formed in an entrance part of the optical fiber, and is widely formed as to be distanced in the longitudinal direction of the optical fiber from the entrance part, and each the optical fiber adheres via an adhesive layer mutually, is provided.

According to another aspect of the present invention, an optical fiber bundle comprising a plurality of optical fibers which can be inserted in a tubular tissue in an organism, each the optical fiber comprising: a glass core layer; a covering core layer which covers the glass core layer; and a cladding layer which covers the covering core layer and includes a plurality of apertural areas having constant width in a longitudinal direction of the optical fiber, wherein an arrangement density of the apertural area increases as to be distanced in the longitudinal direction of the optical fiber from an entrance part of the optical fiber, and each the optical fiber adheres via an adhesive layer mutually, is provided.

According to another aspect of the present invention, an optical fiber bundle comprising a plurality of optical fibers which can be inserted in a tubular tissue in an organism, each the optical fiber comprising: a glass core layer; a covering core layer which covers the glass core layer; and a cladding layer which covers the covering core layer and includes a spiral apertural area in a longitudinal direction of the optical fiber, wherein an arrangement density of the apertural area increases as to be distanced in the longitudinal direction of the optical fiber from an entrance part of the optical fiber, and each the optical fiber adheres via an adhesive layer mutually, is provided.

### BRIEF DESCRIPTION OF THE DRAWINGS

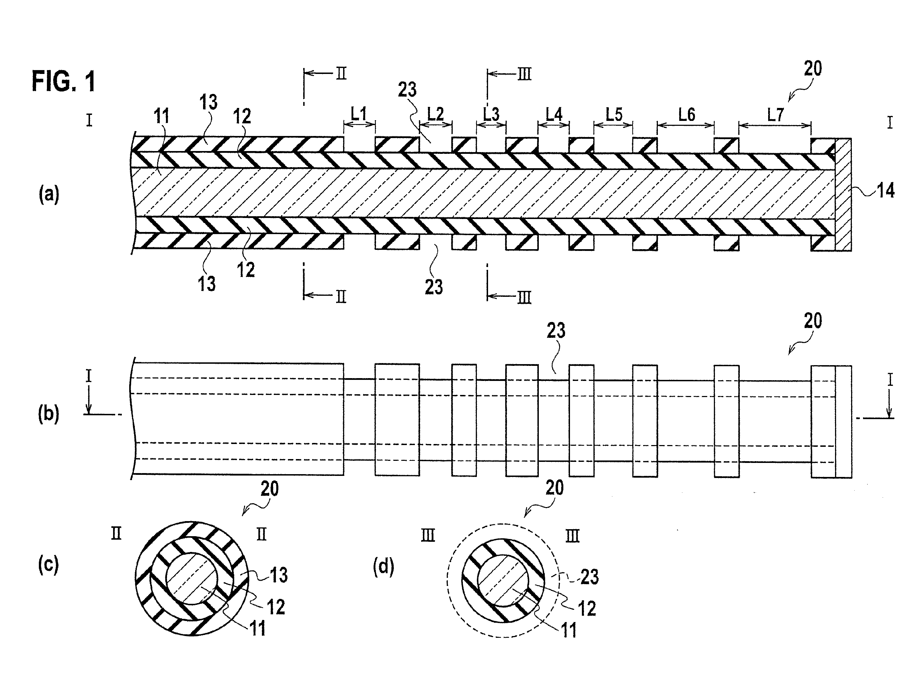

[Fig. 1] An optical fiber according to a first embodiment of the present invention,
   (a) a schematic cross-sectional configuration diagram taken in the line I - I of (b),
   (b) a schematic plan view,
   (c) a schematic cross-sectional configuration diagram taken in the line II - II of (a), and
   (d) a schematic cross-sectional configuration diagram taken in the line III - III of (a).

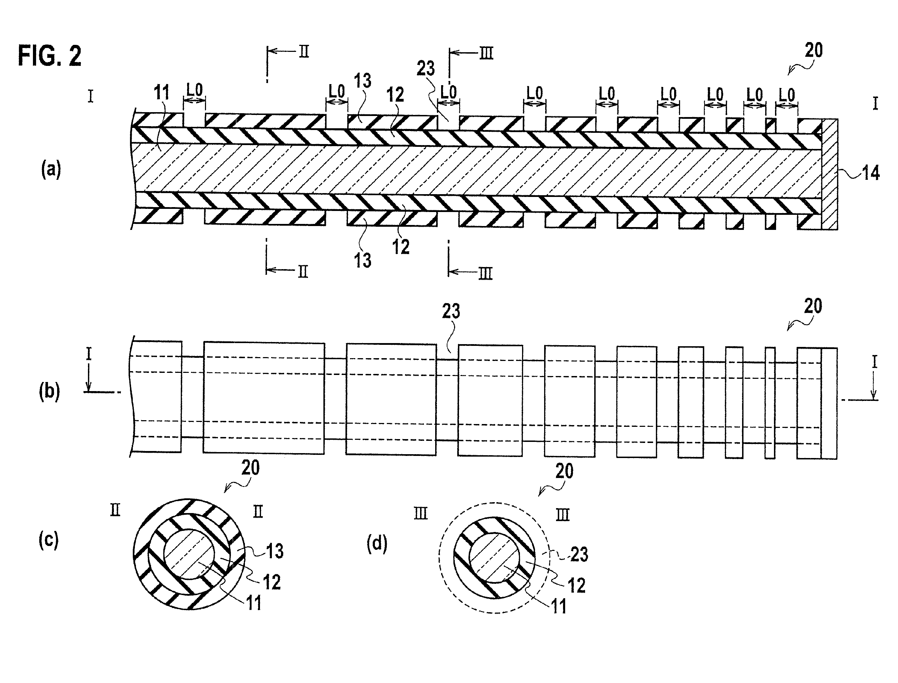

[Fig. 2] An optical fiber according to a second embodiment of the present invention,
   (a) a schematic cross-sectional configuration diagram taken in the line I - I of (b),
   (b) a schematic plan view,
   (c) a schematic cross-sectional configuration diagram taken in the line II - II of (a), and
   (d) a schematic cross-sectional configuration diagram taken in the line III - III of (a).

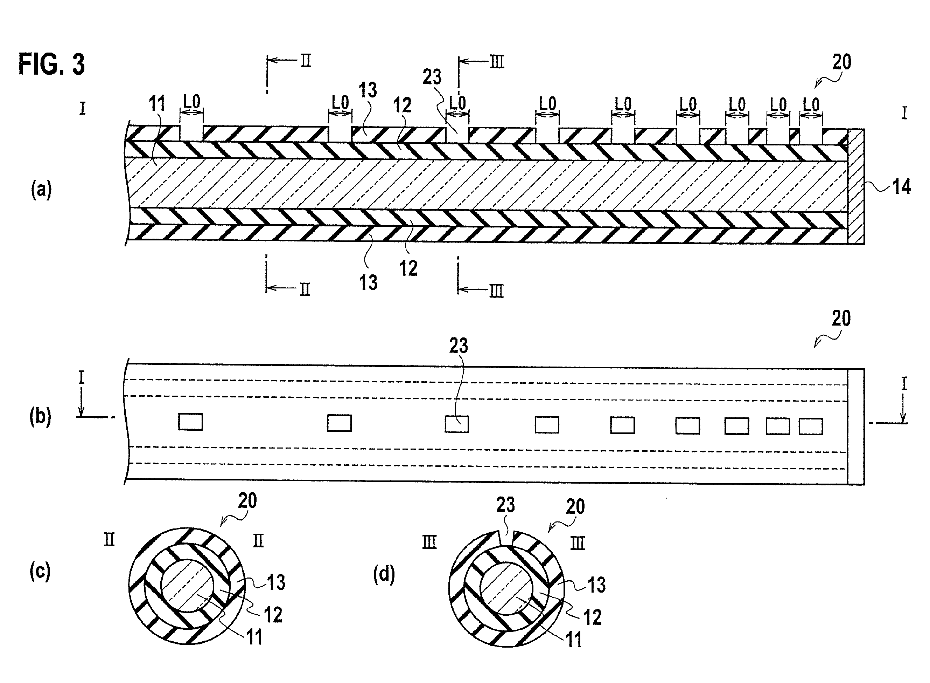

[Fig. 3] An optical fiber according to a third embodiment of the present invention,
   (a) a schematic cross-sectional configuration diagram taken in the line I - I of (b),
   (b) a schematic plan view,
   (c) a schematic cross-sectional configuration diagram taken in the line II - II of (a), and
   (d) a schematic cross-sectional configuration diagram taken in the line III - III of (a).

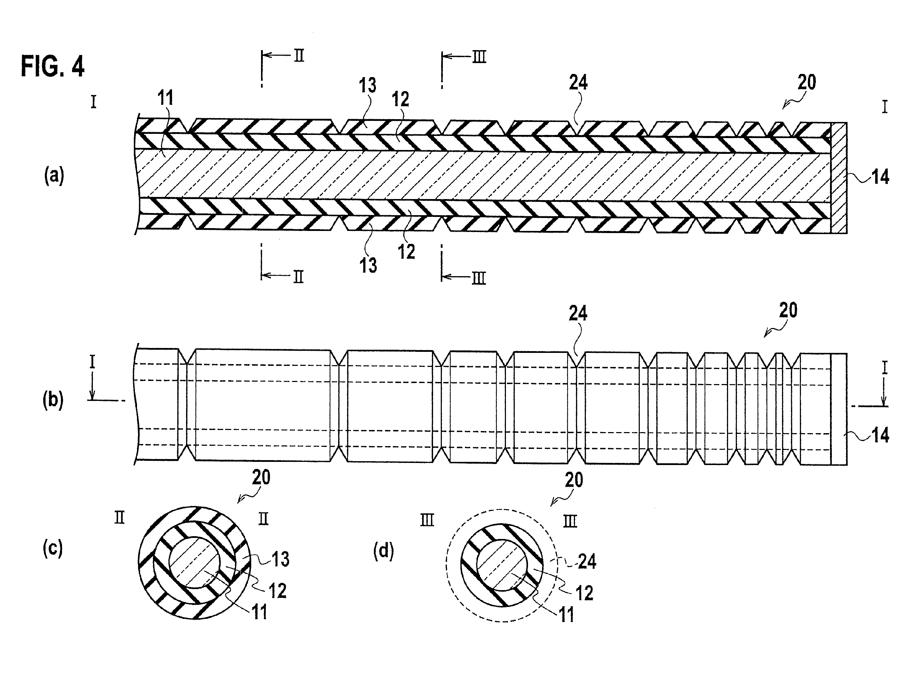

[Fig. 4] An optical fiber according to a fourth embodiment of the present invention,
   (a) a schematic cross-sectional configuration diagram taken in the line I - I of (b),
   (b) a schematic plan view,
   (c) a schematic cross-sectional configuration diagram taken in the line II - II of (a), and
   (d) a schematic cross-sectional configuration diagram taken in the line III - III of (a).
[Fig. 5] An optical fiber according to a fifth embodiment of the present invention,
   (a) a schematic cross-sectional configuration diagram taken in the line I - I of (b), and
   (b) a schematic plan view.
[Fig. 6] An optical fiber according to a sixth embodiment of the present invention,
   (a) a schematic cross-sectional configuration diagram taken in the line I - I of (b), and
   (b) a schematic plan view.
[Fig. 7] A schematic cross-sectional configuration diagram for explaining a status that the optical fiber according to the second embodiment of the present invention is inserted in organic tubular tissue.
[Fig. 8] A schematic cross-sectional configuration diagram taken in the line I - I of Fig. 7.
[Fig. 9] A schematic diagram showing the relation between the light intensity P and length L, in the optical fiber according to the first embodiment of the present invention and an optical fiber according to a comparative example.
[Fig. 10]
   (a) a schematic cross-sectional configuration diagram of the optical fiber according to the first embodiment of the present invention, and
   (b) a schematic cross-sectional configuration diagram of the optical fiber according to a comparative example.

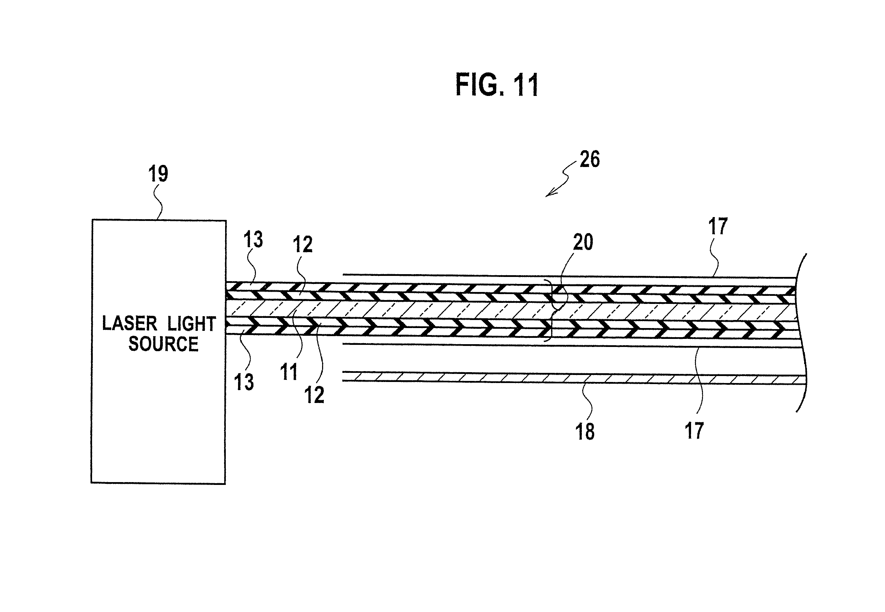

[Fig. 11] A schematic configuration diagram of an optical fiber device which applies the optical fiber according to the first to sixth embodiments of the present invention.

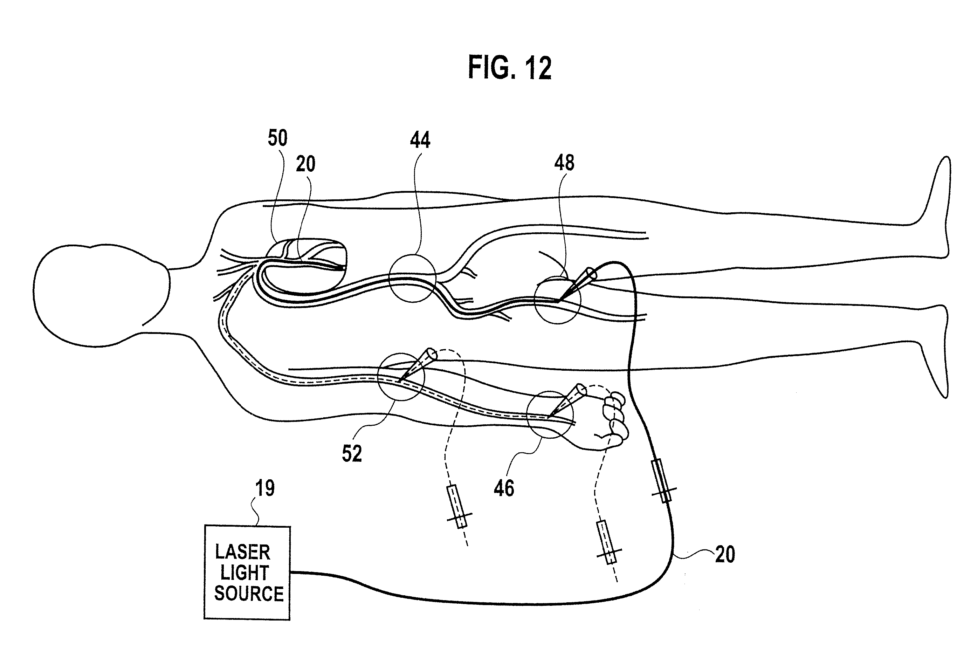

[Fig. 12] A schematic diagram for explaining an aspect of a cardiac catheter operation or an examination using an optical fiber device which applies the optical fiber according to the first to sixth embodiments of the present invention.

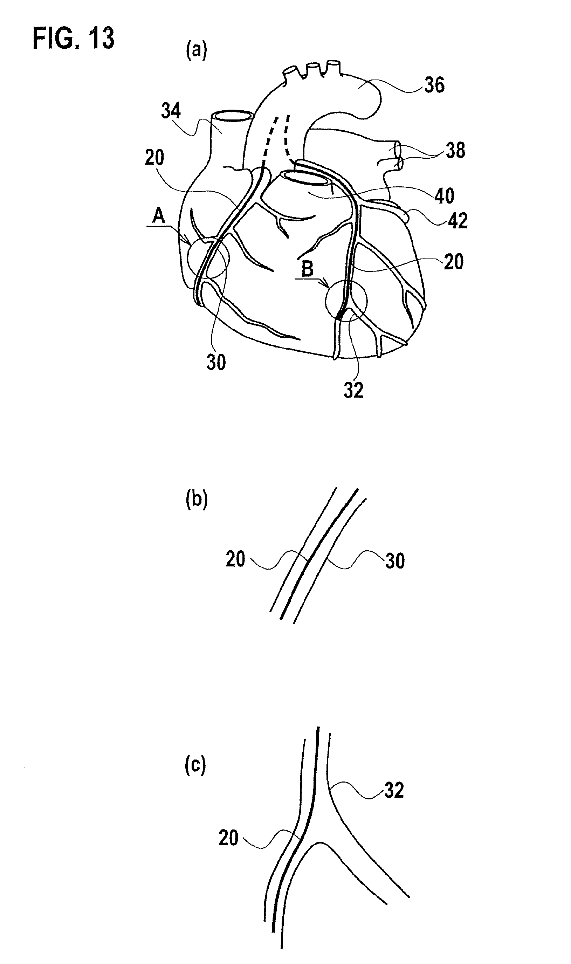

[Fig. 13] In the cardiac catheter operation or the examination using the optical fiber device which applies the optical fiber according to the first to sixth embodiments of the present invention,
   (a) a figure for explaining an aspect that the optical fiber according to the first to sixth embodiments of the present invention is inserted in the cardiac coronary artery part,
   (b) an enlarged drawing of the part of A, and
   (c) an enlarged drawing of the part of B.

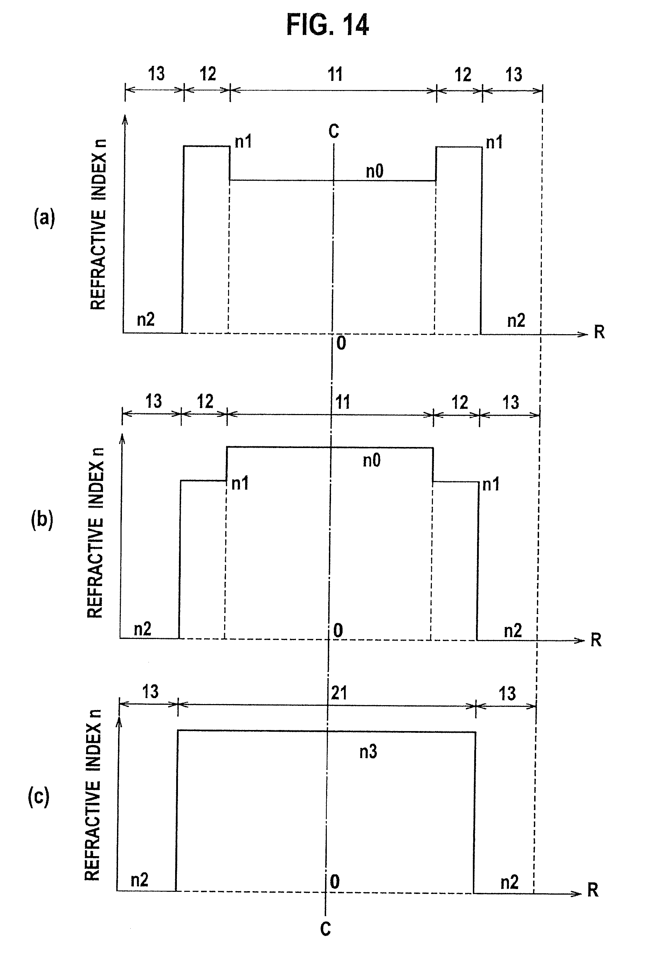

[Fig. 14] An example of a refractive index profile of the optical fiber related to the first to sixth embodiments of the present invention,
   (a) an example whose refractive index n1 of a covering core layer is high, for a refractive index n0 of glass core layer,
   (b) an example whose refractive index n1 of a covering core layer is low, for a refractive index n0 of glass core layer, and
   (c) an example whose refractive index n3 of plastic core layer is the flat.

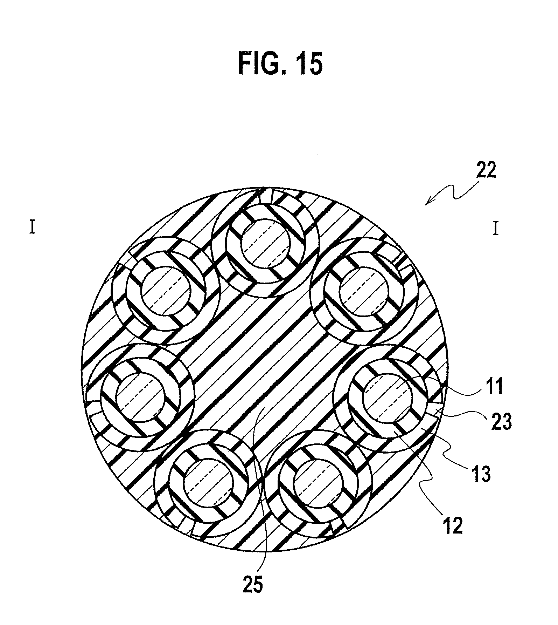

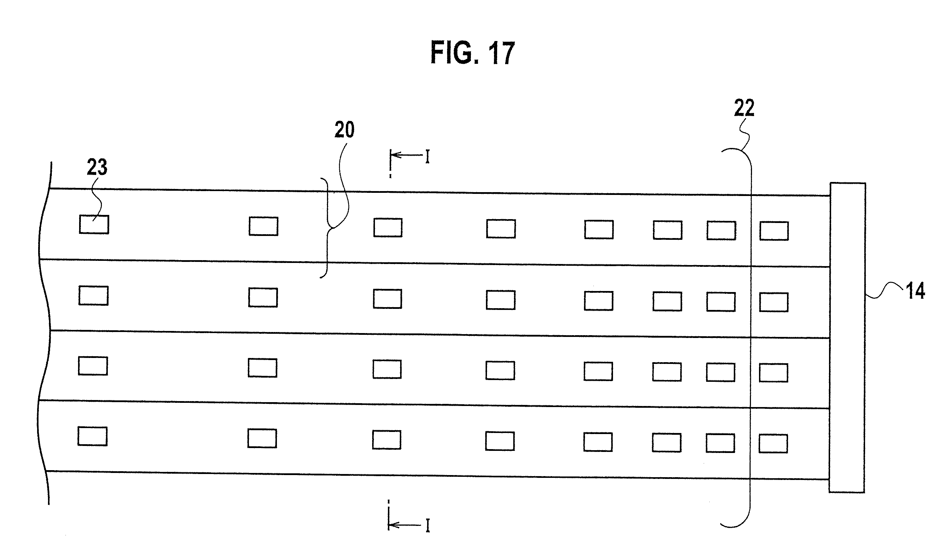

[Fig. 15] A schematic cross-sectional configuration diagram which is the optical fiber according to the eighth embodiment of the present invention, and is taken in the line I - I of Fig. 17 showing an example which composes the optical fiber according to the third embodiment of the present invention into an optical fiber bundle.

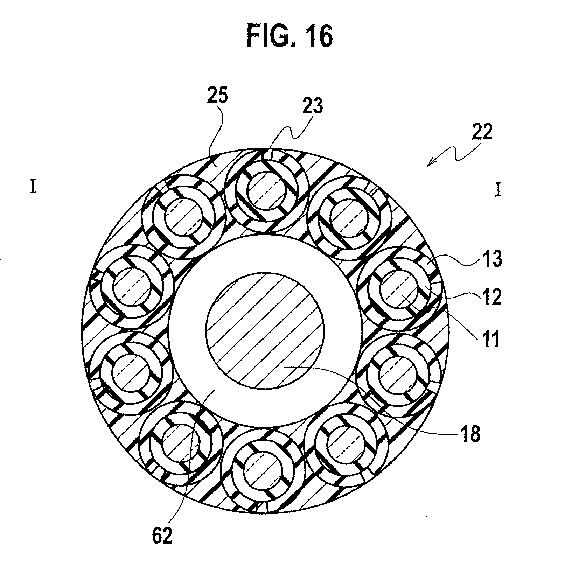

[Fig. 16] A schematic cross-sectional configuration diagram of an optical fiber according to a modified example of the eighth embodiment of the present invention.
[Fig. 17] A schematic plan view which is the optical fiber according to the eighth embodiment of the present invention, and composes the optical fiber according to the third embodiment of the present invention into the optical fiber bundle.

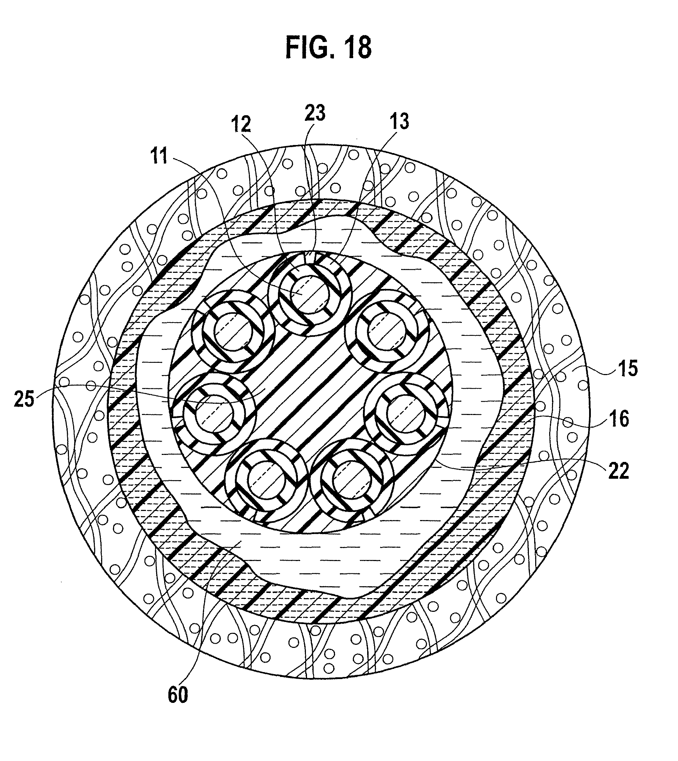

[Fig. 18] A schematic cross-sectional configuration diagram for explaining the status that the optical fiber bundle shown in Fig. 15 is inserted in organic tubular tissue.

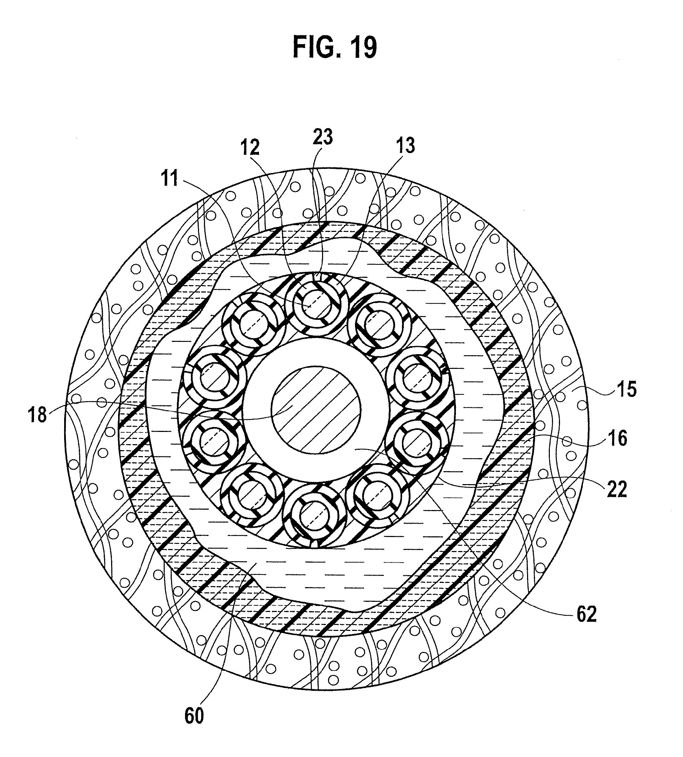

[Fig. 19] A schematic cross-sectional configuration diagram for explaining the status that the optical fiber bundle shown in Fig. 16 is inserted in organic tubular tissue.

### BEST MODE FOR CARRYING OUT THE INVENTION

Next, an embodiment of the invention is described with reference to drawings. In the description of the following drawings, the identical or similar reference numeral is attached to the identical or similar part. However, a drawing is schematic and it should care about differing from an actual thing. Of course, the part from which the relation and ratio of a mutual size differ also in mutually drawings is included.

The embodiment shown in the following exemplifies the device and method for materializing the technical idea of this invention, and the technical idea of this invention does not specify placement of each component parts, etc. as the following. The technical idea of this invention can add various change in scope of claims.

Moreover, an optical fiber, an optical fiber device, and an optical fiber bundle according to the embodiments of the present invention can provide the means for removing a lesion part of internal wall of organic tubular tissue by using together with PDT (Photodynamic Therapy) or PDD (Photodynamic Diagnosis).

Furthermore, the optical fiber, the optical fiber device, and the optical fiber bundle according to the embodiments of the present invention can provide the diagnosing method and the operating method for removing the lesion part of the organic tubular tissue internal wall, by using together with PDT (Photodynamic Therapy) or PDD (Photodynamic Diagnosis).

In addition, in the explanation of the embodiments of the present invention, the tubular tissue indicates a blood vessel, a pancreatic duct, a bile duct, a trachea, a mammary gland, lactiferous ducts, a urinary duct, etc., for example. In particular, all the blood vessels that include a part which the infarction and obstruction generate by atheroma, the thrombus, etc. about the blood vessel not only including a cardiac coronary artery but including a brain artery, a pulmonary vein, carotid arteries, and an artery and a vein of abdominal part and hand and foot, are applicable.

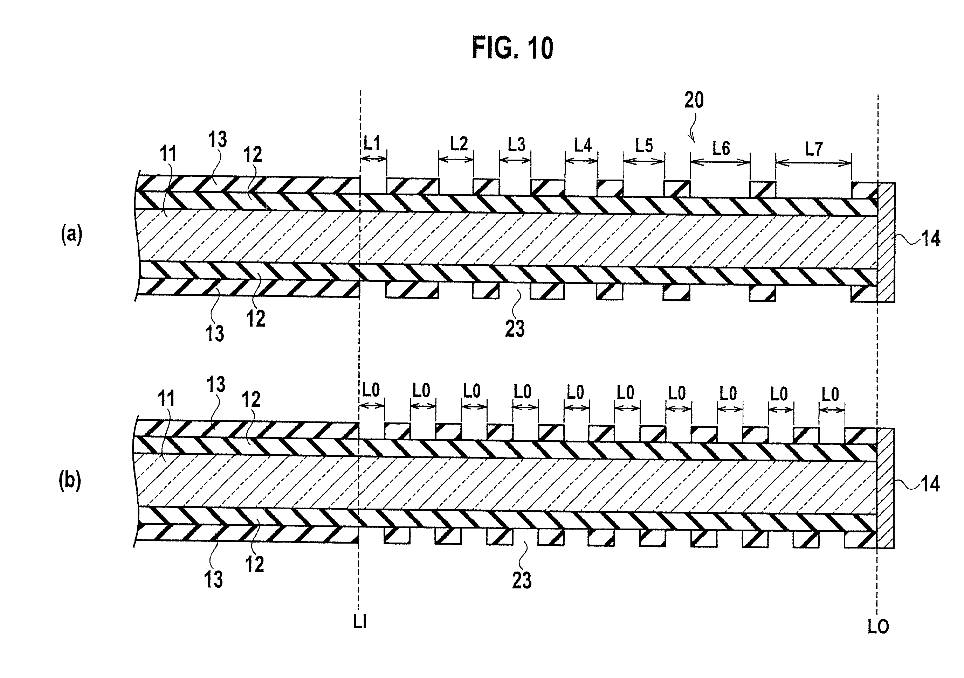

Moreover, in the embodiments of the present invention, as shown in Fig. 10, the outlet part LO of the optical fiber is a part by which the reflection mirror 14 is placed, and as shown in Fig. 10, the entrance part LI of the optical fiber is a part by which the first apertural area 23 is placed in view of the laser light source.

Moreover, in the embodiments of the present invention, the arrangement density of the apertural area 23 is defined by the overall length of the apertural area 23 per unit-length in the longitudinal direction of the optical fiber 20. That the arrangement density of the apertural area 23 is low means that the rate of the length of the apertural area 23 per unit-length in the longitudinal direction is relatively low. That the arrangement density of the apertural area 23 is high means that the rate of the length of the apertural area 23 per unit-length in the longitudinal direction of the optical fiber 20 is relatively high.

### [First Embodiment]

An optical fiber 20 according to the first embodiment of the present invention is expressed as shown in Fig. 1. Fig. 1(a) is a schematic cross-sectional configuration diagram taken in the line I - I of Fig. 1(b), Fig. 1(b) is a schematic plan view of the optical fiber 20, Fig. 1(c) is a schematic cross-sectional configuration diagram taken in the line II - II of Fig. 1(a), and Fig. 1(d) shows a schematic cross-sectional configuration diagram taken in the line III - III of Fig. 1(a).

As shown in Fig. 1, the optical fiber 20 according to the first embodiment of the present invention is an optical fiber which can be inserted in a tubular tissue in an organism, and includes: a glass core layer 11; a covering core layer 12 which covers the glass core layer 11; and a cladding layer 13 which covers the covering core layer 12 and includes a plurality of apertural areas 23 in the longitudinal direction of the optical fiber 20. In the longitudinal direction of the optical fiber 20, the width L1-L7 of the apertural area 23 is in the size relation of L1<L2<L3<L4<L5<L6<L7, and it is narrowly formed in the entrance part of the optical fiber 20 and is widely formed as it is distanced in the longitudinal direction of the optical fiber 20 from the entrance part.

In this case, in the first embodiment of the present invention, as shown in Fig. 10, the outlet part LO of the optical fiber is a part by which a reflection mirror 14 is placed, and as shown in Fig. 10, the entrance part LI of the optical fiber is a part by which the first apertural area 23 is placed in view of the laser light source.

Moreover, in the optical fiber 20 according to the first embodiment of the present invention, the apertural area 23 has ring shape, as shown in Fig. 1.

### (Relation between Light Intensity and Apertural Area Placement)

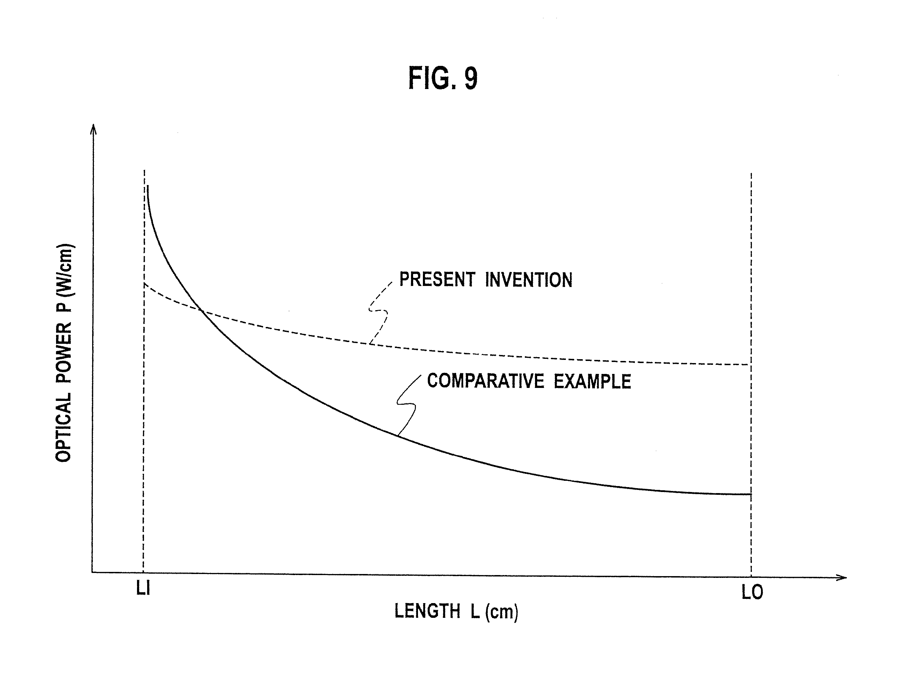

In the optical fiber according to the first embodiment of the present invention and an optical fiber according to a comparative example, the relation between the light intensity P and length L is expressed, as shown in Fig. 9. A schematic cross section structure of the optical fiber 20 according to the first embodiment of the present invention corresponding to Fig. 9 is expressed as shown in Fig. 10(a), and a schematic cross section structure of the optical fiber according to the comparative example is expressed as shown in Fig. 10(b).

In the optical fiber according to the comparative example, as shown in Fig. 10(b), the width L0 of the apertural area 23 is a given width, and is placed at equal intervals. As shown by the full line of Fig. 9, the light power P (W/cm) is strong in the entrance part LI of the optical fiber, and the light power P (W/cm) is rapidly decreased as it shifts in the direction of length L of the longitudinal direction of the optical fiber towards the outlet part LO of the optical fiber. In the optical fiber according to the comparative example, this is because almost all light power P (W/cm) will leak near the entrance part LI of the optical fiber since the width LO of the apertural area 23 is a given width and is placed at equal intervals, as shown in Fig. 10(b).

As compared with this, in the optical fiber 20 according to the first embodiment of the present invention, as shown in Fig. 10(a), since the width L1-L7 of the apertural area 23 is in the size relation of L1<L2<L3<L4<L5<L6<L7, and is narrowly formed in the entrance part LI of the optical fiber 20, and is formed so that it may become large gradually towards the outlet part LO from the entrance part LI, the light power P (W/cm) is not decreased rapidly, it decreases gradually or the essential almost flat output characteristic is obtained, as it shifts in the direction of length L of the longitudinal direction of the optical fiber 20. In the optical fiber 20 according to the first embodiment of the present invention, as shown in Fig. 10(a), this is because leakage of light power P (W/cm) near the entrance part LI of the optical fiber 20 is suppressed since the width of the apertural area 23 is formed narrowly near the entrance part LI of the optical fiber 20, and is formed so that it may become large gradually towards outlet part LO from the entrance part LI.

As for the blood vessels, such as a coronary artery, when inserting the optical fiber, there is the trend for the inside diameter to become thin as it goes to the end part although the inside diameter of the entrance part is generally thick. In the optical fiber 20 related to the first embodiment of the present invention, by adjusting the length L of the optical fiber 20 part by which the a plurality of apertural areas 23 are placed according to the length of the blood vessels, such as the coronary artery, and the relation of the inside diameter, it is also possible to set up the relation between the light power P (W/cm) and the length L so that it may decrease gradually or the essential almost flat output characteristic may be obtained, as shown by the dotted line of Fig. 9.

### (Refractive Index Profile)

Fig. 14 shows an example of the refractive index profile of the optical fiber 20 according to the first embodiment of the present invention, Fig. 14(a) shows an example whose refractive index n1 of the covering core layer 12 is high for the refractive index n0 of the glass core layer 11, Fig. 14(b) shows an example whose refractive index n1 of the covering core layer 12 is low for the refractive index n0 of the glass core layer 11, and Fig. 14(c) shows an example refractive index n3 of the plastic core layer 21 is flat.

In the optical fiber 20 according to the first embodiment of the present invention, the profile of the applicable refractive index n is expressed as shown in Fig. 14(a) to Fig. 14(c). In Fig. 14, central-axis C-C of the longitudinal direction of the optical fiber 20 is set up as a central axis of the axis of coordinates, and the profile of the refractive index n of the direction of radius R is shown.

In the optical fiber 20 according to the first embodiment of the present invention, as shown in Fig. 14(a), the refractive index n0 of the glass core layer 11 is low set up a little rather than the refractive index n1 of the covering core layer 12. Thus, by setting up the refractive index n1 of the covering core layer 12 higher than the refractive index n0 of the glass core layer 11, there is the effect that the light which flows through the optical fiber 20 becomes easy to perform the light guide of the covering core layer 12, thereby making easy to leak from the apertural area 23 placed at the cladding layer 13. Moreover, the refractive index n3 of the cladding layer 13 is set up lower than the refractive index n0 of the glass core layer 11. As a typical numerical value, n0=1.4585, n1=1.46 to 1.53, and n3=1.38 to 1.4 are preferable, for example.

When silica is used for the glass core layer 11, for example, the refractive index n0=1.4585 is obtained. As a material of the covering core layer 12, when the silicone resin is used, for example, the refractive index n1=1.52 is obtained. Moreover, when epoxy acrylate based resin is used, for example, the refractive index n1=1.543 is obtained. Moreover, when ethyl carbamate acrylate based resin is used, for example, the refractive index n1=1.49 to 1.52, 1.510 to 1.513, or 1.490 to 1.540 is obtained.

On the other hand, as a material of the cladding layer 13, when the silicone resin for silicone clad is used, for example, the refractive index n2=1.41 is obtained. Moreover, when fluoridation acrylate based resin is used, for example, the refractive index n2=1.38 or 1.40 is obtained. Moreover, when ethyl carbamate acrylate based resin is used, for example, the refractive index n2=1.470 to 1.490 is obtained.

The refractive index n1 of the covering core layer 12 and the refractive index n2 of the cladding layer 13 can be used combining the refractive index of the above-mentioned material suitably. The selection of the refractive index and material of the glass core layer 11, the covering core layer 12, and the cladding layer 13 is not limited above. It is selectable suitably in other plastic materials.

On the other hand, in the optical fiber 20 according to the first embodiment of the present invention, as shown in Fig. 14(b), the refractive index n0 of the glass core layer 11 can also be set up higher than the refractive index n1 of the covering core layer 12. Thus, by setting up the refractive index n1 of the covering core layer 12 lower than the refractive index n0 of the glass core layer 11, the light which flows through the optical fiber 20 becomes easy to perform the light guide of the glass core layer 11 relatively, can perform the light guide of the longer distance in the glass core layer 11 of the optical fiber 20, and can be made easy to leak also from the apertural area 23 placed at the longer distance in the longitudinal direction of the optical fiber 20 from the entrance part in light. Therefore, in the case of the optical fiber 20 which has the refractive index profile of Fig. 14(b), it is applicable at the use which sets up the length of the optical fiber 20 long.

Furthermore, in the optical fiber 20 according to the first embodiment of the present invention, as shown in Fig. 14(c), it is also possible to use a plastic core layer 21 as the core layer. The refractive index profile of the refractive index n3 of the plastic core layer 21 has the flat profile, as shown in Fig. 14(c). It can apply to the case where the power level of the light which penetrates through the optical fiber 20 is relatively low as the optical fiber 20 using the plastic core layer 21 which has the flat refractive index profile.

Moreover, in the optical fiber 20 according to the first embodiment of the present invention, as shown in Fig. 1, a reflection mirror 14 is further placed in the trailer of the optical fiber 20. The metallic materials, such as the nickel (Ni), the chromium (Cr), and the aluminum (Al), or the multilayer film can be used for the reflection mirror 14, for example.

In addition, the diameter of the optical fiber according to the first embodiment of the present invention including the glass core layer 11, the covering core layer 12, and the cladding layer 13 is about 0.01 to several mm, preferable, is about 0.01 to 1 mm, and is selected according to the inside diameter of the organic tubular tissue to apply.

### (Relation between Light Wavelength and Medicine)

The optical fiber 20 according to the first embodiment of the present invention can apply PDT (Photodynamic Therapy) by inserting in the organic tubular tissue, and can recover the lesion of the internal wall of organic tubular tissue completely. The organism introduces the photosensitizer, the thrombus part which the medicine concentrated selectively is illuminated with the laser beam of the specific wavelength by using that the medicine of the photosensitizer concentrates selectively on the thrombus part which the atheroma, the cholesterol, etc. deposit, and the photochemical reaction can dissolve and remove the thrombus part.

When Antrin is used as the photosensitizer, for example, about 732 nm is the proper center wavelength as the wavelength of the laser beam. When Photofrin or ALA is used as the photosensitizer, for example, about 630 nm is the proper center wavelength as the wavelength of the laser beam. When Foscan is used as the photosensitizer, for example, about 652 nm is the proper center wavelength as the wavelength of the laser beam. When LS11 is used as the Photosensitizer, for example, about 654 nm is the proper center wavelength as the wavelength of the laser beam.

Moreover, the far-infrared wavelength can also be used from infrared with low dispersion absorption loss by the hemoglobin, etc. in the blood. For example, the laser beam, which has a wavelength of 1.0 to 1.3 µm (preferable near 1.1 µm), can be used. Alternatively, the laser beam, which has the wavelength of 1.5 to 2.0 µm (preferable near 1.6 µm), can also be used.

It is also possible to provide a diagnosing method which grips the broadening range of the lesion part using the medicine of the photosensitizer concentrating on the lesion part selectively, by using the PDD (Photodynamic Diagnosis) together with such the PDT (photodynamic therapy). That is, after gripping the broadening range of the lesion part in the organic tubular tissue, etc. using the PDD (Photodynamic Diagnosis), the optical fiber 20 according to the first embodiment of the present invention is introduced in the organic tubular tissue, the lesion part is illuminated with the laser beam of the proper wavelength by using the medicine of the photosensitizer concentrating on the lesion part selectively according to the broadening range of the lesion part, the thrombus part adhering to the internal wall of the organic tubular tissue can be dissolved and removed, and the lesion can be recovered completely.

The applied dose of the photosensitizer is about 2.5 mg, for example, and the power levels of the laser beam are about 150 J/cm·f⁻¹, for example (where f denotes a repeat frequency of the laser beam). When f is 1 kHz, the power level becomes about 150 mJ/cm. Such the laser beam illuminates about 500 sec, and then the thrombus part adhering to the internal wall of organic tubular tissue, etc. can be dissolved and removed.

In addition, the optical power level is not limited to the above-mentioned numerical value range, and may use weaker power according to the use. According to the length of the target organism's tubular tissue, and the optical power level in which the photosensitizer performs the photoreaction, the quality of the material of the glass core layer 11 can also be changed into plastics from silica. Moreover, it is also possible to omit the covering core layer 12 and to compose only the glass core layer 11 or only the plastic core layer according to the length of the target organism's tubular tissue and the optical power level in which the photosensitizer performs the photoreaction.

As for the blood vessels, such as a coronary artery, when inserting the optical fiber, there is the trend for the inside diameter to become thin as it goes to the end part although the inside diameter of the entrance part is generally thick. In the optical fiber 20 according to the first embodiment of the present invention, since the relation between the light power P (W/cm) and the length L is decreased gradually or the essential almost flat output characteristic is obtained, the PDT (Photodynamic therapy) is applied to the whole coronary artery, and the thrombus part 16, etc. adhering to the internal wall of the coronary artery can be dissolved and removed, by adjusting the length L of the optical fiber 20 part having the apertural area 23 to about 20 to 30 cm even if the length of the tubular tissue 15 is about 20 to 30 cm which is the length of the cardiac coronary artery, for example.

### [Second Embodiment]

An optical fiber 20 according to a second embodiment of the present invention is expressed as shown in Fig. 2. Fig. 2 (a) is a schematic cross-sectional configuration diagram taken in the line I - I of Fig. 2(b), Fig. 2(b) is a schematic plan view of the optical fiber 20, Fig. 2(c) is a schematic cross-sectional configuration diagram taken in the line II - II of Fig. 2(a), and Fig. 2(d) shows a schematic cross-sectional configuration diagram taken in the line III - III of Fig. 2(a).

As shown in Fig. 2, the optical fiber 20 according to the second embodiment of the present invention is an optical fiber which can be inserted in a tubular tissue in an organism, and includes: a glass core layer 11; a covering core layer 12 which covers the glass core layer 11; and a cladding layer 13 which covers the covering core layer 12 and includes a plurality of apertural areas 23 having the given width L0 in the longitudinal direction of the optical fiber 20. The arrangement density of the apertural area 23 increases as it is distanced in the longitudinal direction of the optical fiber 20 from the entrance part of the optical fiber 20.

Moreover, in the second embodiment of the present invention, the arrangement density of the apertural area 23 is defined by the overall length of the apertural area 23 per unit-length in the longitudinal direction of the optical fiber 20. That the arrangement density of the apertural area 23 is low in the entrance part of the optical fiber 20 means that the rate of the length of the apertural area 23 per unit-length in the longitudinal direction is relatively small in the entrance part of the optical fiber 20. That the arrangement density of the apertural area 23 is high means the rate of the length of the apertural area 23 per unit-length in the longitudinal direction of the optical fiber 20 is relatively large.

Moreover, in the optical fiber 20 according to the second embodiment of the present invention, the apertural area 23 has ring shape, as shown in Fig. 2.

Moreover, in the optical fiber 20 according to the second embodiment of the present invention, as shown in Fig. 2, a reflection mirror 14 is placed in the trailer of the optical fiber 20. The metallic materials, such as the nickel (Ni), the chromium (Cr), and the aluminum (Al), or the multilayer film can be used for the reflection mirror 14, for example.

### (Refractive Index Profile)

As well as the first embodiment, the profile of the refractive index n applicable with the optical fiber 20 according to the second embodiment of the present invention is expressed as shown in Fig. 14(a) to Fig. 14(c). Moreover, selection of the material of each part and the value of the refractive index n are the same as that of the first embodiment.

### (Relation between Light Intensity and Apertural Area Placement)

Also in the optical fiber 20 according to the second embodiment of the present invention, the relation between the light intensity P and the length L is expressed as the dotted line in Fig. 9 shows, as well as the optical fiber 20 according to the first embodiment of the present invention.

Also in the optical fiber 20 according to the second embodiment of the present invention, since the arrangement density of the apertural area 23 is relatively low in the entrance part LI of the optical fiber 20 and increases as it shifts from the entrance part LI to the direction of length L of the longitudinal direction of the optical fiber 20 towards the outlet part LO, the light power P (W/cm) is not decreased rapidly, it decreases gradually, or the essential almost flat output characteristic is obtained.

### (Relation between Light Wavelength and Medicine)

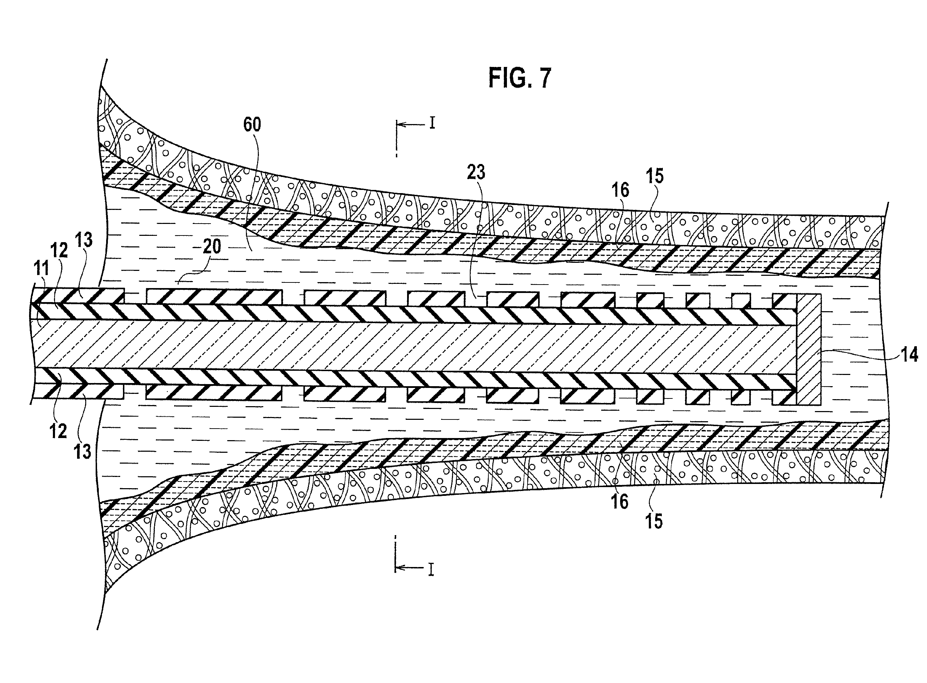

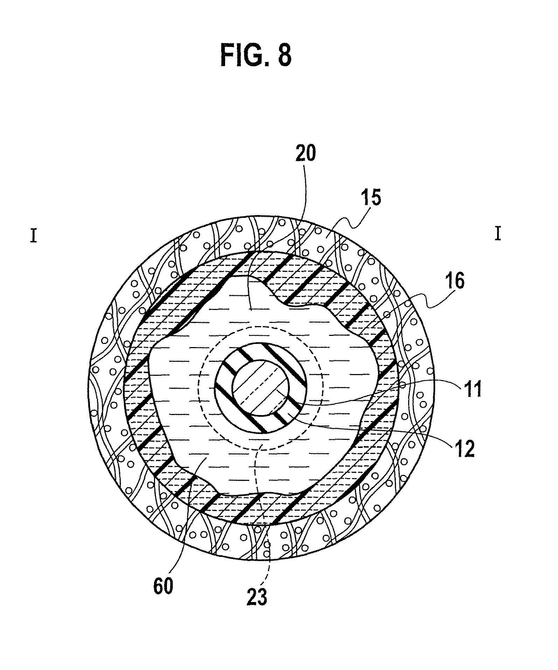

Fig. 7 is a schematic cross-sectional configuration diagram for explaining the status that the optical fiber 20 according to the second embodiment of the present invention is inserted into a fluid medium 60 in an organic tubular tissue 15. In this case, the fluid medium 60 is blood etc. , for example. Moreover, Fig. 8 is a schematic cross-sectional configuration diagram taken in the line I - I of Fig. 7. As shown in Fig. 7 and Fig. 8, since deposition formation of the thrombus part 16 is performed at the internal wall of the organic tubular tissue 15, the thrombus part 16 adhering to the internal wall of the organic tubular tissue 15 can be dissolved and removed by also inserting the optical fiber 20 according to the second embodiment of the present invention into the organic tubular tissue 15, and applying the PDT (photodynamic therapy). The organism introduces the photosensitizer, the thrombus part 16 which the medicine concentrated selectively is illuminated with the laser beam of the specific wavelength by using that the medicine of the photosensitizer concentrates selectively on the thrombus part 16 which the atheroma, the cholesterol, etc. deposit, and the photochemical reaction can dissolve and remove the thrombi, such as the plaque layer.

The relation between the photosensitizer and the wavelength of the laser beam is the same as that of the explanation in the first embodiment.

That the PDD (Photodynamic Diagnosis) is used together with the PDT (Photodynamic Therapy), and the thrombi, such as the plaque layer, adhering to the internal wall of organic tubular tissue can be dissolved and removed is also that of the same as the explanation in the first embodiment.

Also about the applied dose of the photosensitizer, and the power level of the laser beam, it is the same as that of the explanation in the first embodiment.

### [Third Embodiment]

An optical fiber 20 according to a third embodiment of the present invention is expressed as shown in Fig. 3. Fig. 3(a) is a schematic cross-sectional configuration diagram taken in the line I - I of Fig. 3(b), Fig. 3(b) is a schematic plan view of the optical fiber 20, Fig. 3(c) is a schematic cross-sectional configuration diagram taken in the line II - II of Fig. 3(a), and Fig. 3(d) shows a schematic cross-sectional configuration diagram taken in the line III - III of Fig. 3(a).

As shown in Fig. 3, the optical fiber 20 according to the third embodiment of the present invention is an optical fiber which can be inserted in the tubular tissue in the organism, and includes: a glass core layer 11; a covering core layer 12 which covers the glass core layer 11; and a cladding layer 13 which covers the covering core layer 12 and includes a plurality of apertural areas 23 having constant width in the longitudinal direction of the optical fiber 20. The arrangement density of the apertural area 23 increases as it is distanced in the longitudinal direction from the entrance part of the optical fiber 20.

Moreover, in the optical fiber 20 according to the third embodiment of the present invention, the apertural area 23 has rectangular shape, as shown in Fig. 3.

Moreover, in the optical fiber 20 according to the third embodiment of the present invention, as shown in Fig. 3, a reflection mirror 14 is placed in the trailer of the optical fiber 20. The metallic materials, such as the nickel (Ni), the chromium (Cr), and the aluminum (Al), or the multilayer film can be used for the reflection mirror 14, for example.

Moreover, in the optical fiber 20 according to the third embodiment of the present invention, as shown in Fig. 3, since the apertural area 23 has rectangular shape and the light is emitted only from the window part in which the apertural area 23 exists, when using the optical fiber 20 inserting in the organic tubular tissue, the optical fiber 20 may be used rotating around the central axis of the longitudinal direction.

### (Refractive Index Profile)

As well as the first embodiment, the profile of the refractive index n applicable with the optical fiber 20 according to the third embodiment of the present invention is expressed as shown in Fig. 14(a) to Fig. 14(c). Moreover, selection of the material of each part and the value of the refractive index n are the same as that of the first embodiment.

### (Relation between Light Intensity and Apertural Area Placement)

Also in the optical fiber 20 according to the third embodiment of the present invention, the relation between the light intensity P and the length L is expressed as the dotted line in Fig. 9 shows, as well as the optical fiber 20 according to the first embodiment of the present invention.

Also in the optical fiber 20 according to the third embodiment of the present invention, since the arrangement density of the apertural area 23 is relatively low in the entrance part LI of the optical fiber 20 and increases as it shifts from the entrance part LI to the direction of length L of the longitudinal direction of the optical fiber 20 towards the outlet part LO, the light power P (W/cm) is not decreased rapidly, it decreases gradually, or the essential almost flat output characteristic is obtained.

### (Relation between Light Wavelength and Medicine)

The thrombi, such as the plaque layer, adhering to the internal wall of organic tubular tissue can be dissolved and removed by inserting also the optical fiber 20 according to the third embodiment of the present invention in the organic tubular tissue and applying the PDT (photodynamic therapy), as well as the optical fiber 20 according to the first embodiment. The organism introduces the photosensitizer, the thrombus part which the medicine concentrated selectively is illuminated with the laser beam of the specific wavelength using the medicine of the photosensitizer concentrating on the thrombus part which atheroma, the cholesterol, etc. deposited selectively, and thereby the photochemical reaction can dissolve and remove the thrombus part.

The relation between the photosensitizer and the wavelength of the laser beam is the same as that of the explanation in the first embodiment.

That the PDD (Photodynamic Diagnosis) is used together with the PDT (Photodynamic Therapy) and the thrombus part adhering to the internal wall of organic tubular tissue can be dissolved and removed is also that of the same as the explanation in the first embodiment.

Also about the applied dose of the photosensitizer, and the power level of the laser beam, it is the same as that of the explanation in the first embodiment.

### [Fourth Embodiment]

An optical fiber 20 according to a fourth embodiment of the present invention is expressed as shown in Fig. 4. Fig. 4 (a) is a schematic cross-sectional configuration diagram taken in the line I - I of Fig. 4(b), Fig. 4(b) is a schematic plan view of the optical fiber 20, Fig. 4(c) is a schematic cross-sectional configuration diagram taken in the line II - II Fig. 4(a), and Fig. 4(d) shows a schematic cross-sectional configuration diagram taken in the line III - III of Fig. 4 (a).

As shown in Fig. 4, the optical fiber 20 according to the fourth embodiment of the present invention is an optical fiber which can be inserted in the tubular tissue in the organism, and includes: a glass core layer 11; a covering core layer 12 which covers the glass core layer 11; and a cladding layer 13 which covers the covering core layer 12 and includes a plurality of trench apertural areas (notches) 24 in the longitudinal direction of the optical fiber 20. The arrangement density of the trench apertural area (notch) 24 is relatively low in the entrance part of the optical fiber 20 and increases as it is distanced in the longitudinal direction of the optical fiber 20 from the entrance part.

Moreover, in the optical fiber 20 according to the fourth embodiment of the present invention, as shown in Fig. 4, the trench apertural area 24 has ring shape.

Moreover, in the optical fiber 20 related to the fourth embodiment of the present invention, the trench apertural area 24 has the notch shape of the V-shaped groove, as shown in Fig. 4.

Moreover, in the optical fiber 20 according to the fourth embodiment of the present invention, as shown in Fig. 4, a reflection mirror 14 is placed in the trailer of the optical fiber 20. The metallic materials, such as the nickel (Ni), the chromium (Cr), and the aluminum (Al), or the multilayer film can be used for the reflection mirror 14, for example.

### (Refractive Index Profile)

As well as the first embodiment, the profile of the refractive index n applicable with the optical fiber 20 according to the fourth embodiment of the present invention is expressed as shown in Fig. 14(a) to Fig. 14(c). Moreover, selection of the material of each part and the value of the refractive index n are the same as that of the first embodiment.

### (Relation between Light Intensity and Apertural Area Placement)

Also in the optical fiber 20 according to the fourth embodiment of the present invention, the relation between the light intensity P and the length L is expressed as the dotted line in Fig. 9 shows, as well as the optical fiber 20 according to the first embodiment of the present invention.

Also in the optical fiber 20 according to the fourth embodiment of the present invention, since the arrangement density of the trench apertural area 24 is relatively low in the entrance part LI of the optical fiber 20 and increases as it shifts from the entrance part LI to the direction of length L of the longitudinal direction of the optical fiber 20 towards the outlet part LO, the light power P (W/cm) is not decreased rapidly, it decreases gradually, or the essential almost flat output characteristic is obtained.

The thrombi adhering to the internal wall of organic tubular tissue can be dissolved and removed by inserting also the optical fiber 20 according to the fourth embodiment of the present invention in the organic tubular tissue and applying the PDT (photodynamic therapy), as well as the optical fiber 20 according to the first embodiment. The organism introduces the photosensitizer, the thrombus part which the medicine concentrated selectively is illuminated with the laser beam of the specific wavelength by using that the medicine of the photosensitizer concentrates selectively on the thrombus part which the atheroma, the cholesterol, etc. deposit, and thereby the photochemical reaction can dissolve and remove the thrombus part, such as the plaque layer.

The relation between the photosensitizer and the wavelength of the laser beam is the same as that of the explanation in the first embodiment.

That the PDD (Photodynamic Diagnosis) is used together with the PDT (Photodynamic Therapy) and the thrombus part adhering to the internal wall of organic tubular tissue can be dissolved and removed is also that of the same as the explanation in the first embodiment.

Also about the applied dose of the photosensitizer, and the power level of the laser beam, it is the same as that of the explanation in the first embodiment.

### [Fifth Embodiment]

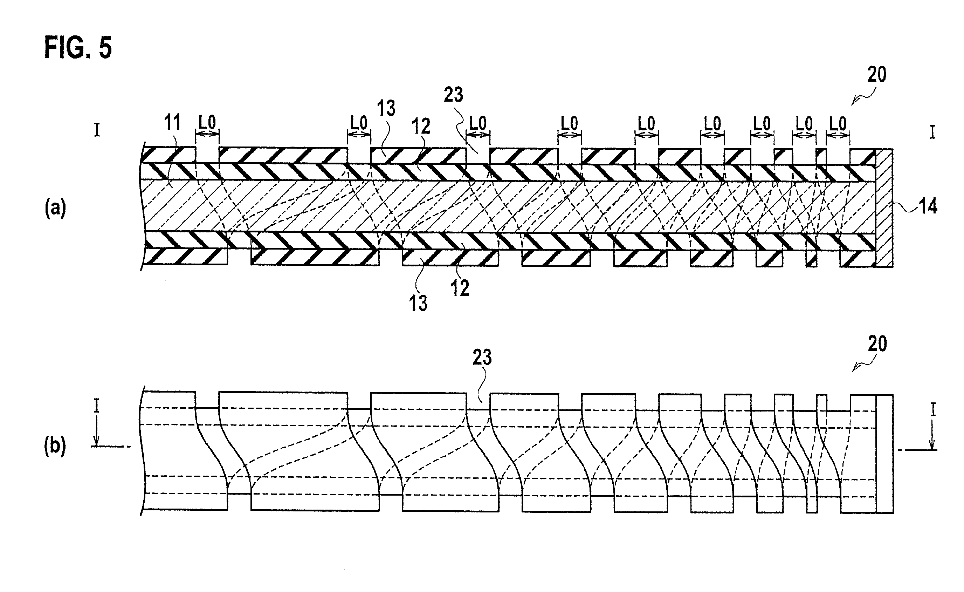

An optical fiber 20 according to a fifth embodiment of the present invention is expressed as shown in Fig. 5. Fig. 5(a) is a schematic cross-sectional configuration diagram taken in the line I - I of Fig. 5(b), and Fig. 5(b) shows a schematic plan view.

As shown in Fig. 5, the optical fiber 20 according to the fifth embodiment of the present invention is an optical fiber 20 which can be inserted in the tubular tissue in the organism, and includes: a glass core layer 11; a covering core layer 12 which covers the glass core layer 11; and a cladding layer 13 which covers the covering core layer 12 and includes a spiral apertural area 23 in the longitudinal direction of the optical fiber 20. The arrangement density of the apertural area 23 is relatively low in the entrance part of the optical fiber 20 and increases as it is distanced in the longitudinal direction of the optical fiber 20 from the entrance part.

In the optical fiber 20 according to the fifth embodiment of the present invention, as shown in Fig. 5, the apertural area 23 has the given width L0 in the longitudinal direction of the optical fiber 20.

Moreover, in the optical fiber 20 according to the fifth embodiment of the present invention, as shown in Fig. 5, a reflection mirror 14 is placed in the trailer of the optical fiber 20. The metallic materials, such as the nickel (Ni), the chromium (Cr), and the aluminum (Al), or the multilayer film can be used for the reflection mirror 14, for example.

### (Refractive Index Profile)

As well as the first embodiment, the profile of the refractive index n applicable with the optical fiber 20 according to the fifth embodiment of the present invention is expressed as shown in Fig. 14(a) to Fig. 14(c). Moreover, selection of the material of each part and the value of the refractive index n are the same as that of the first embodiment.

### (Relation between Light Intensity and Apertural Area Placement)

Also in the optical fiber 20 according to the fifth embodiment of the present invention, the relation between the light intensity P and the length L is expressed as the dotted line in Fig. 9 shows, as well as the optical fiber 20 according to the first embodiment of the present invention.

Also in the optical fiber 20 according to the fifth embodiment of the present invention, since the arrangement density of the apertural area 23 is relatively low in the entrance part LI of the optical fiber 20 and increases as it shifts from the entrance part LI to the direction of length L of the longitudinal direction of the optical fiber 20 towards the outlet part LO, the light power P (W/cm) is not decreased rapidly, it decreases gradually, or the essential almost flat output characteristic is obtained.

### (Relation between Light Wavelength and Medicine)

The thrombi adhering to the internal wall of organic tubular tissue can be dissolved and removed by inserting also the optical fiber 20 according to the fifth embodiment of the present invention in the organic tubular tissue and applying the PDT (photodynamic therapy), as well as the optical fiber 20 according to the first embodiment. The organism introduces the photosensitizer, the thrombus part which the medicine concentrated selectively is illuminated with the laser beam of the specific wavelength using the medicine of the photosensitizer concentrating on the thrombus part which atheroma, the cholesterol, etc. deposited selectively, and thereby the photochemical reaction can dissolve and remove the thrombus part.

The relation between the photosensitizer and the wavelength of the laser beam is the same as that of the explanation in the first embodiment.

That the PDD (Photodynamic Diagnosis) is used together with the PDT (Photodynamic Therapy) and the thrombus part adhering to the internal wall of organic tubular tissue can be dissolved and removed is also that of the same as the explanation in the first embodiment.

Also about the applied dose of the photosensitizer, and the power level of the laser beam, it is the same as that of the explanation in the first embodiment.

### [Sixth Embodiment]

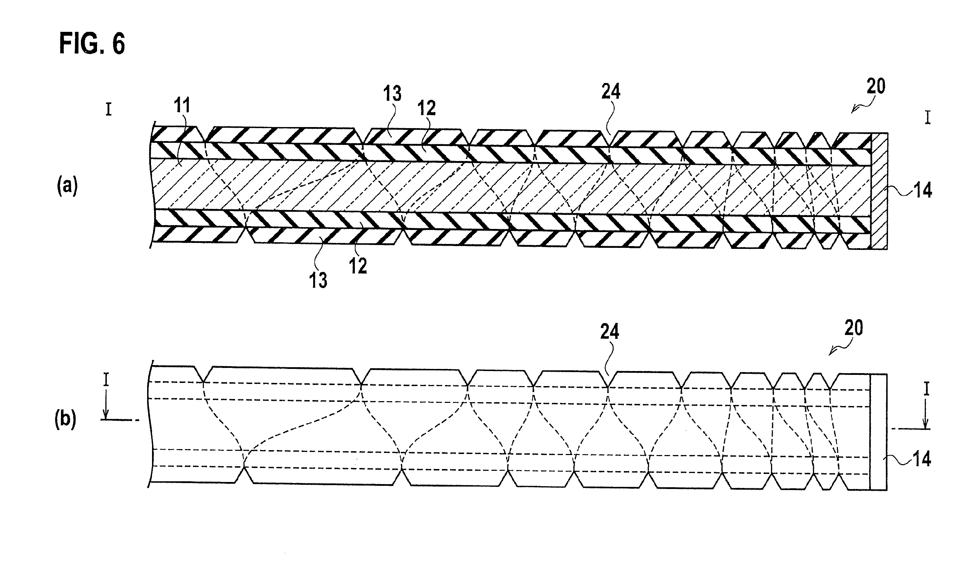

The optical fiber 20 according to a sixth embodiment of the present invention is expressed as shown in Fig. 6. Fig. 6 (a) is a schematic cross-sectional configuration diagram taken in the line I - I of Fig. 6(b), and Fig. 6(b) shows a schematic plan view.

As shown in Fig. 6, the optical fiber 20 according to the sixth embodiment of the present invention is an optical fiber 20 which can be inserted in the tubular tissue in the organism, and includes: a glass core layer 11; a covering core layer 12 which covers the glass core layer 11; and a cladding layer 13 which covers the covering core layer 12 and includes a spiral trench apertural area 24 in the longitudinal direction of the optical fiber 20. The arrangement density of the trench apertural area 24 is relatively low in the entrance part of the optical fiber 20 and increases as it is distanced in the longitudinal direction of the optical fiber 20 from the entrance part.

Moreover, in the optical fiber 20 according to the sixth embodiment of the present invention, the trench apertural area 24 has the notch shape of the V-shaped groove, as shown in Fig. 6.

Moreover, in the optical fiber 20 according to the sixth embodiment of the present invention, as shown in Fig. 6, a reflection mirror 14 is placed in the trailer of the optical fiber 20. The metallic materials, such as the nickel (Ni), the chromium (Cr), and the aluminum (Al), or the multilayer film can be used for the reflection mirror 14, for example.

### (Refractive Index Profile)

As well as the first embodiment, the profile of the refractive index n applicable with the optical fiber 20 according to the sixth embodiment of the present invention is expressed as shown in Fig. 14(a) to Fig. 14(c). Moreover, selection of the material of each part and the value of the refractive index n are the same as that of the first embodiment.

### (Relation between Light Intensity and Apertural Area Placement)

Also in the optical fiber 20 according to the sixth embodiment of the present invention, the relation between the light intensity P and the length L is expressed as the dotted line in Fig. 9 shows, as well as the optical fiber 20 according to the first embodiment of the present invention.

Also in the optical fiber 20 according to the sixth embodiment of the present invention, since the arrangement density of the trench apertural area 24 is relatively low in the entrance part LI of the optical fiber 20 and increases as it shifts from the entrance part LI to the direction of length L of the longitudinal direction of the optical fiber 20 towards the outlet part LO, the light power P (W/cm) is not decreased rapidly, it decreases gradually, or the essential almost flat output characteristic is obtained.

### (Relation between Light Wavelength and Medicine)

The thrombi adhering to the internal wall of organic tubular tissue can be dissolved and removed by inserting also the optical fiber 20 according to the sixth embodiment of the present invention in the organic tubular tissue and applying the PDT (photodynamic therapy), as well as the optical fiber 20 according to the first embodiment. The organism introduces the photosensitizer, the thrombus part which the medicine concentrated selectively is illuminated with the laser beam of the specific wavelength using the medicine of the photosensitizer concentrating on the thrombus part which atheroma, the cholesterol, etc. deposited selectively, and thereby the photochemical reaction can dissolve and remove the thrombus part.

The relation between the photosensitizer and the wavelength of the laser beam is the same as that of the explanation in the first embodiment.

That the PDD (Photodynamic Diagnosis) is used together with the PDT (Photodynamic Therapy) and the thrombus part adhering to the internal wall of organic tubular tissue can be dissolved and removed is also that of the same as the explanation in the first embodiment.

Also about the applied dose of the photosensitizer, and the power level of the laser beam, it is the same as that of the explanation in the first embodiment.

### [Seventh Embodiment]

Fig. 11 shows a schematic configuration diagram of an optical fiber device 26 which applies the optical fiber 20 according to the first to sixth embodiments of the present invention. In Fig. 11, since the detailed configuration of the apertural area of the optical fiber 20 is the same as that of the optical fiber 20 according to the first to sixth embodiments of the present invention, illustration is omitted.

The optical fiber device 26 according to the seventh embodiment of the present invention includes: a laser light source 19; an optical fiber 20 which is connected to the laser light source 19 and is the optical fiber according to the first to sixth embodiments of the present invention; a guidewire 18 by which adjacent disposition is performed to the optical fiber 20; and a catheter 17 which inserts the optical fiber 20.

In the optical fiber device 26 according to the seventh embodiment of the present invention, the guidewire 18 by which adjacent disposition is performed to the optical fiber 20 may be inserted into the catheter 17 which inserts the optical fiber 20.

The optical fiber device 26 according to the seventh embodiment of the present invention is simple for the configuration of the whole device, and the light weight and the miniaturization are easy also for the laser light source by applying the semiconductor laser etc.

The cardiac catheter operation or examination using the optical fiber device 26 which applies the optical fiber 20 according to the first to sixth embodiments of the present invention is schematically expressed as shown in Fig. 12. The optical fiber 20 connected to the laser light source 19 is inserted from the femoral artery 48, and is made to reach in the heart 50 through the aorta abdominalis 44. Similarly the optical fiber 20 connected to the laser light source 19 is inserted from the radial artery 46 or the elbow part artery 52, and may be made to reach in the heart 50.

In the cardiac catheter operation or examination using the optical fiber device 26 which applies the optical fiber 20 according to the first to sixth embodiments of the present invention, an aspect that the optical fiber according to the first to sixth embodiments of the present invention is inserted in the cardiac coronary artery part is schematically expressed as shown in Fig. 13(a). Fig. 13(b) is an enlarged drawing of a part of A of Fig. 13(a), and Fig. 13(c) is an enlarged drawing of a part of B of Fig. 13(a).

As shown in Fig. 13(a), the optical fiber 20 which reached in the heart 50 via the aorta 36 near the heart 50 is inserted into the right coronary artery 30, the left anterior descending artery 32, or the left coronary artery circumflex artery 42, for example. Fig. 13(b) shows an example by which the optical fiber 20 is inserted into the right coronary artery 30. Fig. 13(c) shows an example by which the optical fiber 20 is inserted into the left anterior descending artery 32. Moreover, according to the use, the optical fiber 20 may be inserted into the large vein 34, the pulmonary vein 38, or the pulmonary artery 40.

In the above-mentioned explanation, although the case where the optical fiber 20 according to the first to sixth embodiments of the present invention is applied was explained as an example, the optical fiber bundle 22 according to an eighth embodiment of the present invention described later is also applicable, as shown in Fig. 15 to Fig. 19.

### [Eighth Embodiment]

Fig. 15 shows an optical fiber according to the eighth embodiment of the present invention, and shows a schematic cross-sectional configuration diagram which composed the optical fiber 20 shown in the third embodiment of the present invention in the optical fiber bundle. Moreover, Fig. 16 shows a modified example of an optical fiber according to the eighth embodiment of the present invention. Moreover, Fig. 17 shows the optical fiber according to the eighth embodiment of the present invention, and shows a schematic plan view which composes the optical fiber 20 shown in the third embodiment of the present invention in the optical fiber bundle. Both of Fig. 15 and Fig. 16 correspond to a schematic cross-sectional configuration diagram taken in the line I - I of Fig. 17. In the optical fiber according to the modified example of the eighth embodiment of the present invention shown in Fig. 16, a schematic plan view is similarly shown to be Fig. 17.

Fig. 18 shows a schematic cross-sectional configuration diagram for explaining the status that the optical fiber bundle 22 shown in Fig. 15 is inserted into the fluid medium 60 of the organic tubular tissue 15. Moreover, Fig. 19 shows a schematic cross-sectional configuration diagram for explaining the status that the optical fiber bundle 22 shown in Fig. 16 is inserted into the fluid medium 60 of the organic tubular tissue 15. In this case, the fluid medium 60 is blood etc.

As shown in Fig. 15, the optical fiber according to the eighth embodiment of the present invention composes the optical fiber bundle 22, and is composed of a plurality of optical fibers which can be inserted in the tubular tissue in the organism. Each optical fiber includes: a glass core layer 11; a covering core layer 12 which covers the glass core layer 11; and a cladding layer 13 which covers the covering core layer 12 and includes a plurality of apertural areas 23 having constant width in the longitudinal direction of the optical fiber. The arrangement density of the apertural area 23 is relatively low in the entrance part of the optical fiber and increases as it is distanced in the longitudinal direction of the optical fiber from the entrance part, and each optical fiber of each other is adhered via the adhesive layer 25 to make it as the bundle.

Each optical fiber which composes the optical fiber bundle 22 according to the eighth embodiment of the present invention is not limited to the optical fiber 20 according to the third embodiment of the present invention, and can all apply the optical fiber 20 explained in the first to sixth embodiments of the present invention. Furthermore, according to the use, it may combine a plurality of kinds of optical fibers 20 explained in the first to sixth embodiments.

The optical fiber according to the modified example of the eighth embodiment of the present invention provides the cavity portion 62 of the central part of a plurality of optical fibers with the guidewire 18, as shown in Fig. 16. By adopting the configuration of Fig. 16, it becomes unnecessary to place the guidewire 18 separately to external of the plurality of optical fibers, and thereby the space-saving and the miniaturization of the optical fiber and the optical fiber device can be accelerated.

Moreover, in the optical fiber according to the eighth embodiment and the modified example of the present invention, the apertural area 23 of each optical fiber may be provided with ring shape, the swirl shape, or the notch shape of the V-shaped groove.

Moreover, in the optical fiber according to the eighth embodiment and the modified example of the present invention, as shown in Fig. 17, a reflection mirror 14 is placed in the trailer of the optical fiber bundle 22. The metallic materials, such as the nickel (Ni), the chromium (Cr), and the aluminum (Al), or the multilayer film can be used for the reflection mirror 14, for example.

Moreover, the position of the apertural area 23 of each optical fiber 20 is completed to be placed when composed as the optical fiber bundle 22 in the example shown in Fig. 17, but it is not necessary to complete the position of the apertural area 23, and it may be shifted to be placed.

In addition, the diameter of the optical fiber bundle 22 shown in the eighth embodiment of the present invention is about 0.1 mm to several mm, is about 0.1 mm to about 1 mm preferable, and is selected according to the number of the bundle, and the inside diameter of organic tubular tissue to apply. For example, in the optical fiber bundle applied to the eighth embodiment of the present invention, in the case where the diameter of each optical fiber including the glass core layer 11, the covering core layer 12, and the cladding layer 13 is about 0.1 mm, if the bundle number is 7 as shown in Fig. 15, the diameter of the optical fiber bundle will be about 0.2 mm to about 0.4 mm. When the plastic core layer 21 is used instead of the glass core layer 11, it can form still more thinly. Also when the bundle is applied, it is also possible to be applied as not more than about 0.1 mm, and it can select according to the inside diameter of the organic tubular tissue to apply, the length of the tubular tissue to apply, and the value of the optical power level.

### (Refractive Index Profile)

Also in the optical fiber bundle 22 shown in the eighth embodiment of the present invention, and its modified example, the profile of the refractive index n applicable with each optical fiber 20 is expressed as shown in Fig. 14(a) to Fig. 14(c), as well as the first embodiment. Moreover, selection of the material of each part and the value of the refractive index n are the same as that of the first embodiment.

### (Relation between Light Intensity and Apertural Area Placement)

Also in the optical fiber bundle 22 shown in the eighth embodiment of the present invention, and its modified example, as for each optical fiber 20 shown in Fig. 17, since the arrangement density of the apertural area 23 is relatively low in the entrance part LI of the optical fiber and increases as it shifts from the entrance part LI to the direction of length L of the longitudinal direction of the optical fiber towards the outlet part LO, the light power P (W/cm) is not decreased rapidly, it decreases gradually, or the essential almost flat output characteristic is obtained. Therefore, as for the relation between the light intensity P and the length L, the same characteristics as the characteristics shown by the dotted line in Fig. 9 are obtained, as well as the optical fiber according to the first embodiment of the present invention. Since it is made as the bundle, the light intensity P becomes large the several times to several tens times, for example, according to the number of the optical fiber.

### (Relation between Light Wavelength and Medicine)

As shown in Fig. 18 and Fig. 19, the optical fiber according to the eighth embodiment of the present invention and its modified example, the thrombus part 16 adhering to the internal wall of the organic tubular tissue 15 can be dissolved and removed by inserting in the organic tubular tissue 15 and applying the PDT (photodynamic therapy), as well as the optical fiber according to the first embodiment.

The relation between the photosensitizer and the wavelength of the laser beam is the same as that of the explanation in the first embodiment.

That the PDD (Photodynamic Diagnosis) is used together with the PDT (Photodynamic Therapy), and the thrombus part 16 can be dissolved and removed is also that of the same as the explanation in the first embodiment.

Also about the applied dose of the photosensitizer, and the power level of the laser beam, it is the same as that of the explanation in the first embodiment.

In the optical fiber according to the eighth embodiment of the present invention, since the relation between the light power P (W/cm) and the length L is decreased gradually or the essential almost flat output characteristic is obtained, even if the length of the tubular tissue 15 is about 20 cm to 30 cm which is the length of the cardiac coronary artery, for example, by adjusting the length L of the optical fiber part having the apertural area 23 to about 20 cm to 30 cm, and applying the PDT (Photodynamic therapy) to the whole coronary artery, the thrombus part 16, etc. adhering to the internal wall of the coronary artery can be dissolved and removed.

The tubular tissues 15 with which the optical fiber bundle 22 shown in the eighth embodiment of the present invention is applied is the blood vessel, the pancreatic duct, the bile duct, the trachea, the mammary gland, lactiferous ducts, the urinary duct, etc.

### [OTHER EMBODIMENTS]

The present invention has been described by the first to eighth embodiments mentioned above, as a disclosure including associated description and drawings to be construed as illustrative, not restrictive. With the disclosure, artisan might easily think up alternative embodiments, embodiment examples, or application techniques.

Such being the case, the present invention covers a variety of embodiments, whether described or not. Therefore, the technical scope of the present invention is appointed only by the invention specific matter related appropriate scope of claims from the above-mentioned explanation.

According to the optical fiber, optical fiber device, and optical fiber bundle of the present invention, it is applied to organic tubular tissue, and the lesion region of the internal wall of tubular tissue can be removed selectively.

Moreover, according to the optical fiber, optical fiber device, and optical fiber bundle of the present invention, the photoconductive phaosome in which the light leaks to the side (the direction of the circumference) is made over a certain length of the optical fiber, and the machinery strength for bending stress etc. is fully maintainable while having its structure.

Moreover, according to the optical fiber, optical fiber device, and optical fiber bundle of the present invention, the laser beam of high intensity can be transmitted by using silica for the core of the optical fiber in particular.

## Claims

1. An optical fiber which can be inserted in a tubular tissue in an organism, the optical fiber comprising:
a glass core layer;
a covering core layer which covers the glass core layer; and
a cladding layer which covers the covering core layer and includes a plurality of apertural areas in a longitudinal direction of the optical fiber, wherein
a width of the apertural area in the longitudinal direction of the optical fiber is narrowly formed in an entrance part of the optical fiber, and is widely formed as to be distanced in the longitudinal direction of the optical fiber from the entrance part.

2. An optical fiber which can be inserted in a tubular tissue in an organism, the optical fiber comprising:
a glass core layer;
a covering core layer which covers the glass core layer; and
a cladding layer which covers the covering core layer and includes a plurality of apertural areas having constant width in a longitudinal direction of the optical fiber, wherein
an arrangement density of the apertural area increases as to be distanced in the longitudinal direction of the optical fiber from an entrance part of the optical fiber.

3. The optical fiber according to claim 1 or 2, wherein the apertural area has notch shape.

4. The optical fiber according to claim 1 or 2, wherein the apertural area has ring shape.

5. An optical fiber which can be inserted in a tubular tissue in an organism, the optical fiber comprising:
a glass core layer;
a covering core layer which covers the glass core layer; and
a cladding layer which covers the covering core layer and includes a spiral apertural area in a longitudinal direction of the optical fiber, wherein
an arrangement density of the apertural area increases as to be distanced in the longitudinal direction of the optical fiber from an entrance part of the optical fiber.

6. The optical fiber according to claim 5, wherein the apertural area has notch shape.

7. The optical fiber according to claim 5, wherein the apertural area has a given width.

8. The optical fiber according to any one of claims 1 to 7, wherein
a dielectric constant of the glass core layer is lower than a dielectric constant of the covering core layer.

9. The optical fiber according to any one of claims 1 to 7, wherein
a dielectric constant of the glass core layer is higher than a dielectric constant of the covering core layer.

10. An optical fiber which can be inserted in a tubular tissue in an organism, the optical fiber comprising:
a core layer; and
a cladding layer which covers the core layer and includes a plurality of apertural areas having constant width in a longitudinal direction of the optical fiber, wherein
an arrangement density of the apertural area increases as to be distanced in the longitudinal direction of the optical fiber from an entrance part of the optical fiber.

11. The optical fiber according to any one of claims 1 to 10, wherein
the tubular tissue is one of a blood vessel, a pancreatic duct, a bile duct, a trachea, a mammary gland, a lactiferous duct, and a urinary duct.

12. The optical fiber according to any one of claims 1 to 11, wherein
a reflection mirror is further placed in a trailer of the optical fiber.

13. An optical fiber device comprising:
a laser light source; and
an optical fiber connected to the laser light source, the optical fiber comprising a glass core layer, a covering core layer which covers the glass core layer, and a cladding layer which covers the covering core layer and including a plurality of apertural areas in a longitudinal direction of the optical fiber, a width of the apertural area in the longitudinal direction of the optical fiber is narrowly formed in an entrance part of the optical fiber and is widely formed as to be distanced in the longitudinal direction of the optical fiber from the entrance part, the optical fiber which can be inserted in a tubular tissue in an organism.

14. An optical fiber device comprising:
a laser light source; and
an optical fiber connected to the laser light source, the optical fiber comprising a glass core layer, a covering core layer which covers the glass core layer, and a cladding layer which covers the covering core layer and includes a plurality of apertural areas having constant width in a longitudinal direction of the optical fiber, an arrangement density of the apertural area increases as to be distanced in the longitudinal direction of the optical fiber from an entrance part of the optical fiber, the optical fiber which can be inserted in a tubular tissue in an organism.

15. An optical fiber device comprising:
a laser light source; and
an optical fiber connected to the laser light source, the optical fiber comprising a glass core layer, a covering core layer which covers the glass core layer, and a cladding layer which covers the covering core layer and includes a spiral apertural area in a longitudinal direction of the optical fiber, an arrangement density of the apertural area increases as to be distanced in the longitudinal direction of the optical fiber from an entrance part of the optical fiber, the optical fiber which can be inserted in a tubular tissue in an organism.

16. An optical fiber bundle comprising a plurality of optical fibers which can be inserted in a tubular tissue in an organism,
each the optical fiber comprising:
a glass core layer;
a covering core layer which covers the glass core layer; and
a cladding layer which covers the covering core layer and including a plurality of apertural areas in a longitudinal direction of the optical fiber, wherein
a width of the apertural area in the longitudinal direction of the optical fiber is narrowly formed in an entrance part of the optical fiber, and is widely formed as to be distanced in the longitudinal direction of the optical fiber from the entrance part, and each the optical fiber adheres via an adhesive layer mutually.

17. An optical fiber bundle comprising a plurality of optical fibers which can be inserted in a tubular tissue in an organism,
each the optical fiber comprising:
a glass core layer;
a covering core layer which covers the glass core layer; and
a cladding layer which covers the covering core layer and includes a plurality of apertural areas having constant width in a longitudinal direction of the optical fiber, wherein
an arrangement density of the apertural area increases as to be distanced in the longitudinal direction of the optical fiber from an entrance part of the optical fiber, and each the optical fiber adheres via an adhesive layer mutually.

18. An optical fiber bundle comprising a plurality of optical fibers which can be inserted in a tubular tissue in an organism,
each the optical fiber comprising:
a glass core layer;
a covering core layer which covers the glass core layer; and
a cladding layer which covers the covering core layer and includes a spiral apertural area in a longitudinal direction of the optical fiber, wherein
an arrangement density of the apertural area increases as to be distanced in the longitudinal direction of the optical fiber from an entrance part of the optical fiber, and each the optical fiber adheres via an adhesive layer mutually.

19. The optical fiber bundle according to any one of claims 16 to 18, wherein
a central part of the plurality of optical fibers is further provided with a guidewire.

20. The optical fiber bundle according to any one of claims 16 to 18, wherein
the tubular tissue is one of a blood vessel, a pancreatic duct, a bile duct, a trachea, a mammary gland, a lactiferous duct, and a urinary duct.
